Europäisches Patentamt

(19) European Patent Office

Office européen des brevets

(11) Publication number: **0 205 085**
**B1**

## (12) EUROPEAN PATENT SPECIFICATION

(45) Date of publication of patent specification: **26.07.89**

(51) Int. Cl.⁴: **A 61 M 31/00, B 01 L 3/02**

(21) Application number: **86107562.0**

(22) Date of filing: **04.06.86**

(54) **Disposable applicator.**

(30) Priority: **13.06.85 SE 8502933**

(43) Date of publication of application:
**17.12.86 Bulletin 86/51**

(45) Publication of the grant of the patent:
**26.07.89 Bulletin 89/30**

(84) Designated Contracting States:
**AT BE CH DE FR GB IT LI LU NL SE**

(56) References cited:
**EP-A-0 006 545**
**DE-A-2 653 051**
**DE-B-1 914 754**
**DK-B- 134 308**

(73) Proprietor: **AB LEO**
**Box 941**
**S-251 09 Helsingborg (SE)**

(72) Inventor: **Lundqvist, Mona Karin**
**Brandsberga PL.223**
**S-260 70 Ljungbyhed (SE)**
Inventor: **Malmborg, Bengt Eber**
**Skanegatan 47**
**S-252 52 Helsingborg (SE)**

(74) Representative: **Thylén, Eva**
**c/o AB Leo Patent Department Box 941**
**S-251 09 Helsingborg (SE)**

Courier Press, Leamington Spa, England.

## Description

### Backround of the invention

Prior art: For many medical reasons it is necessary or desirable to apply various medicaments to body cavities. In order to provide a simple method of applying the medicament with a minimum of unpleasantness, the medicaments are often applied with a syringe type applicator. Most often the patient purchases the syringe prefilled with the medicament which has been prescribed at the desired dosage level.

An applicator of this type is disclosed in the European patent application EP-A-0006545. This applicator consists of an expandable cannula provided with a longitudinal cylindrical hollow chamber. The chamber is intended for a metered quantity of a substance to be administered to a patient. Furthermore, at both ends of the chamber there are provided easily removable means. A rod provided with a grip at one end and with a piston conveniently fitting the hollow chamber of the cannula at the other end is arranged such that by pushing the rod into the hollow chamber all the substance contained therein is ejected. This syringe type applicator is intended for single use and has a relatively simple form. It includes, however, several parts and the manufacturing process will therefore be relatively complicated. From this follows that the manufacturing costs that are of critical importance for disposable devices will be relatively high.

A conventionally used applicator for administering medicaments into body cavities is disclosed in the Danish patent 134,308. This applicator comprises a flat container section and a metering chamber in the form of a cannula which can be inserted into, e.g. the nostril. When pressure is applied to the container section the drug is ejected through the cannula. The pressure is applied by the fingers of the user.

This type of applicator has been well accepted and works well in many cases. A general problem with it is, however, the dosing accuracy. In order to obtain dosing accuracy, which is of critical importance for some types of drugs, the drug composition included in the container must have low viscosity. In order to obtain approximately the correct dose of high viscosity compositions, a large excess of the drug composition must be filled into the container. It is, however, generally not accepted by the health authorities that single dose containers have more than a slight excess of the active drug. The ideal situation is of course when the single dose in the container exactly corresponds to the dose that shall be administered. It is thus obvious that this known type of container is unsuitable for administering high viscosity compositions. When low viscosity compositions are administered through this pipette, the problems are less but could not be totally neglected.

### Objects of the invention

An object of the invention is to provide a disposable single dose syringe type applicator for applying medicaments to body cavities.

A second object is to provide a syringe applicator which can be easily manufactured in one piece.

A third object of the invention is to provide an applicator which can be manufactured at a low price.

A fourth object is to provide an applicator which can be used for various types of medicament compositions.

A fifth object is to provide an applicator having high dosing accuracy.

A sixth object of the invention is to provide a prefilled applicator, which, when used, delivers essentially the whole amount of medicament.

### Summary of the invention

The present invention concerns a disposable single dose syringe type applicator in the form of an elongated body. This body includes two integrally joined sections which can be separated by breaking. One section constitutes a medicament reservoir having two openings. The other section is a plunger which is arranged so as to seal one opening of the reservoir which is prefilled with a medicament. Furthermore, the plunger slidably fits to the reservoir in such a way that essentially the whole medicament content of the reservoir is ejected therefrom when the plunger is inserted into the reservoir. At the other opening end of the medicament reservoir there is arranged an easily removable sealing means.

### Detailed description of the invention

A characterizing feature of applicator according to the present invention is that the medicament reservoir and the plunger are manufactured in one piece. When the applicator is used the section constituting the medicament reservoir and the section constituting the plunger are separated by breaking. In this context the term "breaking" includes all ways of taking apart the two integrally joined sections, such as cracking, turning etc.

The elongated body of the applicator is preferably made of a moulded plastic by techniques well known in the art. Suitable manufacturing processes include both injection moulding and blow moulding.

According to a preferred embodiment of the invention the shape of the leading end of the plunger is essentially the same as the shape of the inside of the reservoir, and the largest cross sectional dimensions of the plunger are only slightly smaller than the cross sectional inner dimensions of the reservoir. It is also preferred that the cross sectional dimensions of the plunger and of the outside and inside of the reservoir are relatively uniform.

According to a preferred embodiment essentially the whole plunger is inserted into the reservoir and the shape of the plunger is the same as the shape of the inside of the reservoir. Optionally a grip could be provided at or near one end of the plunger. The lenghth of the plunger (the grip not being included) could be essentially the same as the outer length of the reservoir. Alternatively the plunger could be slightly longer than the outer length of the reservoir. In both cases the length of the plunger inserted into the reservoir should be sufficient to ensure complete ejection of all the medicament from the reservoir, when the plunger is introduced into the reservoir.

According to another embodiment of the invention the plunger is essentially longer than the medicament reservoir and in this case there is provided a means on the plunger which stops the sliding movement of the plunger at a predetermined distance from the leading end of the plunger. This distance corresponds essentially to the outer length of the reservoir in order to ensure complete removal of the medicament from the reservoir when the plunger is inserted. The part of the plunger behind tne stopping means could then serve as a handle. As well understood by the man skilled in the art there can be provided grooves or recesses in the plunger behind the leading end in order to save material. Another way of saving material is to make the plunger hollow, e.g. in the form of a hollow cylinder. The plunger could also be designed with a leading end fitting slidably and exactly to the inside of the reservoir and a rod having smaller cross sectional dimensions. It is prefered that the plunger as well as the inside and outside of the reservoir are cylindrically shaped.

According to another embodiment the plunger and the inside of the reservoir are slightly taper and the opening of the leading end of the applicator, i.e. the end to be inserted into the body cavity, is slightly smaller than the opposite opening of the reservoir.

The opening of the reservoir, which in the elongated body is sealed by the plunger, could have essentially the same cross sectional dimensions as the cross sectional dimensions of the inside of the reservoir. This means that when the plunger is broken off from the section of the elongated body, which constitutes the medicament reservoir, a relatively large, preferably circular, hole is obtained. A large hole could be preferred when the medicament in the reservoir is a solid substance, e.g. a suppository, or when the medicament is present in the form of a liquid of high viscosity such as a gel or an ointment. In this context it should be mentioned that although the applicator according to the invention can be used for administering different kinds of medical compositions such as solutions, ointments, gels, suppositories, vagitories, into different body cavities such as the nose, rectum, vagina, a preferred embodiment concerns an applicator for administering high viscosity liquids or gels including an active substance into the nose.

The active substance can be any substance within human or veterinary medicine which is suitable for nasal administration. Although the applicator according to the invention in the first place is intended for administering medicines to humans and animals it is obvious that it can also be used within other fields, e.g. within the cosmetic and food industry.

When the reservoir is prefilled with a liquid of lower viscosity it might be preferred that the opening provided in the reservoir is smaller to avoid a too rapid administration of the drug. One way of achieving a relatively small hole in the leading end of the applicator or reservoir is to make the leading end tapered.

In order to ensure complete removal of the medicament from the reservoir the leading end of the plunger should be shaped correspondingly. Instead of manufacturing the reservoir so that only one hole is obtained when the plunger is broken off the applicator can be manufactured in such a way that a plurality of smaller holes are provided and "opened" for passage of the medicament when the plunger is broken off. Furthermore, there could also be provided holes in the cylindrical wall of the reservoir.

The other end of the medicament reservoir can be sealed by any conventional sealing means such as a cap, a plug, an adhesive tape, etc.

Brief description of the drawings

Fig. 1 discloses the principle of the invention.

Fig. 2 is a sectional view of an applicator of the invention.

Fig. 3 is a sectional view of another applicator according to the invention.

Fig. 4 is a plan view of the applicator of Fig. 3.

Fig. 5 is a plan view of the applicator of Fig. 4 ready for use.

Specific reference to the drawings

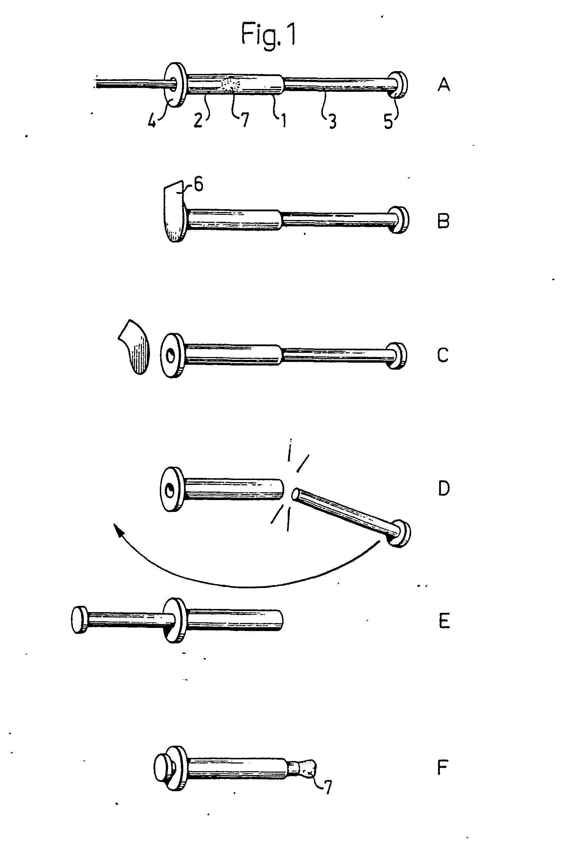

Fig. 1 shows at 1 an elongated body including two sections 2 and 3. A medicament reservoir 2 is sealed at one end by a plunger 3. A grip 5 (not necessary) is provided at the other end of the plunger 3. At the end of the reservoir 2 - not sealed by the plunger 3 - there is provided a flange 4 (4 is preferred but not necessary) allowing a larger surface for the sealing means 6 (in this case an adhesive tape). In position A a medicament 7 is filled into the reservoir 2. The method for filling, sealing, opening both ends of the reservoir 2, introducing the plungers into the reservoir 2 and ejecting the medicament 7 from the reservoir 2 is disclosed in the sequence A-F.

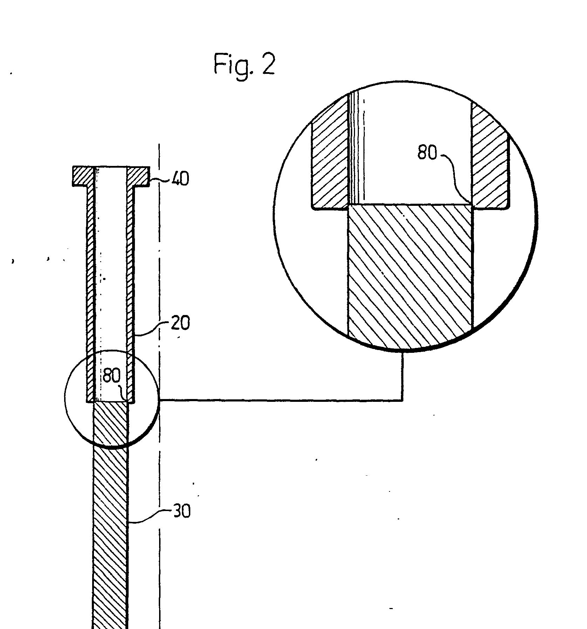

Fig. 2 shows an applicator which is ready for filling. The elongated body comprises the medicament reservoir 20 and a plunger 30 sealing one opening of the reservoir 20. This figure also shows the breakable contact surface at 80.

The material thickness at the breaking notch varies depending on the polymer material used, the manufacturing process and the intended use of the final product. For the embodiment disclosed in figure 2 it could e.g. be 0.1 mm. However, the breakable contact surface should be such that the plunger 30 is firmly joined to the reservoir 20 but could still be comfortably broken off. The configuration of the breakable contact surface can vary and is determined by the shape of the form which is used for the moulding process as is obvious for the man skilled in the art. The applicator disclosed in this figure can be easily and cheaply manufactured by injection moulding.

A special advantage inherent to the embodiment of the invention disclosed in fig. 1 and 2 concerns the hygiene aspect. This can be explained as follows. The end of the plunger which will become the leading end is adjacent to and seals the reservoir opening. When the applicator is to be used the plunger is broken off from the reservoir immediately before use and the leading end (as defined above) is inserted into the reservoir in order to remove the medicine contained therein. By using the applicator in this way the risk of contaminating the medicament is minimal.

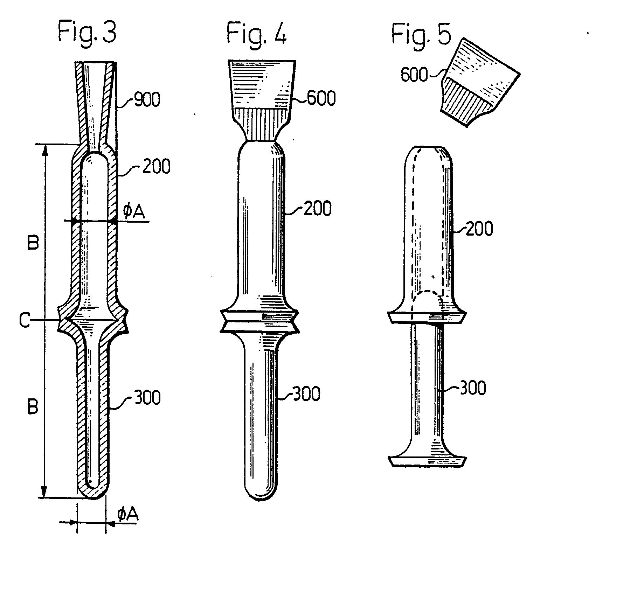

Fig. 3, 4 and 5 show the principle for an applicator manufactured by a blow moulding technique. In the figures 3 and 4 the medicament reservoir 200 and the plunger 300 are integrally joined at C. A is the inner diameter of the reservoir 2DO and the outer diameter of the plunger 300 which is insertable into the reservoir 200 as disclosed in Fig 5.

B indicates the length of the plunger 300 and the length of the medicament reservoir 200.

In Fig. 3 the medicament can be filled into the reservoir 200 through the means 90D which constitutes a sealing means 600 when pressed together as in Fig. 4.

In Fig. 5 the sealing means 600 as well as the plunger 300 has been broken off and plunger 300 introduced into the medicament reservoir 200.

As stated above the fig. 3, 4 and 5 disclose a principle for an applicator made by a blow moulding technique. If the applicator is to be used for large volumes and/or relatively low viscosity liquids (solutions) auxiliary means known to the man skilled in the art must be provided.

**Claims**

1. Disposable, single dose applicator including an elongated body comprising two integrally joined sections which are separable by breaking, one section constituting a medicament reservoir having two openings and the other section constituting a plunger which is arranged so as to seal one opening of said reservoir and which slidably fits to said reservoir in such a way that essentially the whole content of said reservoir is removed therefrom when said plunger is inserted into said reservoir, and an easily removable means which seals the other opening of said reservoir.

2. Applicator according to claim 1 characterized in that the shape of the leading end of said plunger is essentially the same as the shape of the inside of said reservoir, the cross sectional dimensions of the plunger being only slightly smaller than the corresponding dimensions of the inside of said reservoir.

3. Applicator according to claim 1 characterized in that said plunger which is to be inserted into said reservoir has essentially the same shape as the inside of said reservoir.

4. Applicator according to claim 1 characterized in that the part of said plunger which is to be inserted into said reservoir has essentially the same shape as the inside of said reservoir.

5. Applicator according to claim 1 characterized in that the leading end of the plunger has the same shape as and slightly smaller dimensions than the inner dimensions of the opening end of the reservoir adjacent to said leading end sealing said opening.

6. Applicator according to any of the preceding claims in which said elongated body is of circular cross section, the cross sectional diameter of said plunger being only slightly smaller than the inner cross sectional diameter of the medicament reservoir.

7. Applicator according to claim 5 wherein the plunger and inside of the reservoir are cylindrically shaped.

8. Applicator according to claims 1-4, wherein said plunger is essentially as long as said reservoir.

9. Applicator according to claim 1 characterized in that said plunger and the outside of said reservoir have essentially the same shape.

10. Applicator according to any of the preceding claims characterized in that it is adapted for administering a high viscosity liquid including an active substance into the nose.

11. An elongated body comprising two integrally joined sections which are separable by breaking, one section constituting a reservoir having two openings and the other section constituting a plunger which is arranged so as to seal one opening of said reservoir and which slidably fits to said reservoir in such a way that essentially the whole content of said reservoir is removed therefrom when said plunger is inserted into said reservoir.

**Patentansprüche**

1. Einzeldosis-Einwegspritze mit einem länglichen Körper, der aus zwei einteilig miteinander verbundenen Abschnitten besteht, die durch Bruch trennbar sind, wobei der eine Abschnitt einen Medikamentenbehälter mit zwei Öffnungen darstellt und der andere Abschnitt einen Kolben darstellt, der zur Abdichtung einer der Öffnungen des Behälters ausgebildet ist und in den Behälter im Gleitsitz in solcher Weise paßt, daß bei Einschieben des Kolbens in den Behälter im wesentlichender gesamte Inhalt des Behälters aus diesem abgegeben wird, und wobei die andere Öffnung des Behälters mit einem leicht lösbaren Element dicht verschlossen ist.

2. Spritze nach Anspruch 1, dadurch gekennzeichnet, daß die Form des Vorderendes des Kolbens im wesentlichen dieselbe wie die Form des Behälterinneren ist, wobei die Querschnittsabmessungen des Kolbens bloß geringfügig kleiner als die entsprechenden Abmessungen des Behälterinneren sind.

3. Spritze nach Anspruch 1, dadurch gekennzeichnet, daß der in den Behälter einschiebbare Kolben im wesentlichen dieselbe Form wie das Behälterinnere aufweist.

4. Spritze nach Anspruch 1, dadurch gekennzeichnet, daß der in den Behälter einschiebbare Teil des Kolbens im wesentlichen dieselbe Form wie das Behälterinnere aufweist.

5. Spritze nach Anspruch 1, dadurch gekennzeichnet, daß das Vorderende des Kolbens dieselbe Form wie und geringfügig kleinere Abmessungen als die Innenabmessungen des die Öffnung aufweisenden Behälterendes im Bereich des die Öffnung abdichtenden Vorderendes aufweist.

6. Spritze nach den vorhergehenden Ansprüchen, dadurch gekennzeichnet, daß der längliche Körper kreisförmigen Querschnitt aufweist, wobei der Querschnittsdurchmesser des Kolbens bloß geringfügig kleiner als der innere Querschnittsdurchmesser des Medikamentenbehälters ist.

7. Spritze nach Anspruch 5, dadurch gekennzeichnet, daß der Kolben und das Behälterinnere zylindrisch geformt sind.

8. Spritze nach den Ansprüchen 1 bis 4, dadurch gekennzeichnet, daß der Kolben im wesentlichen so lang wie der Behälter ist.

9. Spritze nach Anspruch 1, dadurch gekennzeichnet, daß der Kolben und die Behälteraußenseite im wesentlichen dieselbe Form aufweisen.

10. Spritze nach den vorhergehenden Ansprüchen, dadurch gekennzeichnet, daß sie zur Verabreichung einer hochviskosen Flüssigkeit samt einer aktiven Substanz in die Nase ausgebildet ist.

11. Länglicher Körper, bestehend aus zwei einteilig miteinander verbundenen Abschnitten, die durch Bruch trennbar sind, wobei der eine Abschnitt einen Medikamentenbehälter mit zwei Öffnungen darstellt und der andere Abschnitt einen Kolben darstellt, der zur Abdichtung einer der Öffnungen des Behälters ausgebildet ist und in den Behälter im Gleitsitz in solcher Weise paßt, daß bei Einschieben des Kolbens in den Behälter im wesentlichen der gesamte Inhalt des Behälters aus diesem abgegeben wird.

**Revendications**

1. Applicateur jetable à dose unique comprenant un corps allongé comportant deux sections jointes solidairement pouvant être séparées par rupture, une section constituant un réservoir pour médicaments présentant deux ouvertures et l'autre section constituant un plongeur qui est disposé de manière à obturer de façon étanche une ouverture du réservoir et qui s'adapte de façon coulissante dans ce réservoir de telle manière que sensiblement le volume total contenu dans ce réservoir est extrait de celui-ci lorsque le plongeur est introduit dans le réservoir, et un moyen facilement amovible qui obture de façon étanche l'autre ouverture du réservoir.

2. Applicateur selon la revendication 1, caractérisé en ce que la forme de l'extrémité d'attaque du plongeur est sensiblement la même que la forme de l'intérieur du réservoir, les dimensions en coupe transver sale du plongeur n'étant que légèrement plus petites que les dimensions correspondantes de l'intérieur du réservoir.

3. Applicateur selon la revendication 1, caractérisé en ce que le plongeur qui doit être introduit dans ce réservoir présente sensiblement la même forme que l'intérieur du réservoir.

4. Applicateur selon la revendication 1, caractérisé en ce que la partie du plongeur qui doit être introduite dans le réservoir présente sensiblement la même forme que l'intérieur du réservoir.

5. Applicateur selon la revendication 1, caractérisé en ce que l'extrémité d'attaque du plongeur présente la même forme et des dimensions légèrement plus petites que les dimensions intérieures de l'extrémité d'ouverture du réservoir contiguë à l'extrémité d'attaque obturant l'ouverture de façon étanche.

6. Applicateur selon l'une quelconque des revendications précédentes dans lequel le corps allongé est de section transversale circulaire, le diamètre de la section transversale du plongeur n'étant que légèrement plus petit que le diamètre de section transversale inté rieure du réservoir pour médicaments.

7. Applicateur selon la revendication 5, dans lequel le plongeur et l'intérieur du réservoir sont de configuration cylindrique.

8. Applicateur selon les revendications 1-4, dans lequel le plongeur est sensiblement de même longueur que le réservoir.

9. Applicateur selon la revendication 1, caractérisé en ce que le plongeur et l'extérieur du réservoir présentent sensiblement la même configuration.

10. Applicateur selon l'une quelconque des revendications précédentes, caractérisé en ce qu'il est apte à l'administration d'un liquide à viscosité élevée comprenant une substance active dans les voies nasales.

11. Corps allongé comprenant deux sections jointes solidairement qui sont séparables ou sécables par rupture, une section constituant un réservoir présentant deux ouvertures et l'autre section constituant un plongeur qui est disposé de façon à obturer de façon étanche une ouverture du réservoir et qui s'adapte de façon coulissante sur le réservoir de telle manière que sensiblement la quantité totale contenue dans le réservoir est extraite de celui-ci lorsque le plongeur est introduit dans le réservoir.

# Fig.1

## Fig. 2

Fig. 3

Fig. 4

Fig. 5